# EUROPÄISCHE PATENTSCHRIFT

(11) **EP 0 433 218 B1**
(45) Veröffentlichungstag und Bekanntmachung des Hinweises auf die Patenterteilung: **11.09.1996**
(21) Anmeldenummer: 90810853.3
(22) Anmeldetag: 06.11.1990
(51) Int. Cl.: C07D 253/06, C07D 271/113, C07D 413/04

(54) **Verfahren zur Herstellung von Aminotriazinderivaten**
Method for the preparation of aminotriazin derivatives
Procédé pour la préparation de dérivés de l'aminotriazine

(30) Priorität: 15.11.1989 CH 4107/89
(43) Veröffentlichungstag der Anmeldung: 19.06.1991
(73) Patentinhaber: CIBA-GEIGY AG, 4002 Basel (CH)
(72) Erfinder: Beriger, Ernst, Dr., CH-4123 Allschwil (CH); Kristinsson, Haukur, Dr., CH-4103 Bottmingen (CH)

(56) Entgegenhaltungen:
- EP-A- 0 314 615
- US-A- 4 699 647
- CHEMICAL ABSTRACTS, Band 83, Nr. 15, 13. Oktober 1975, Columbus, Ohio, USA HETZHEIM, ANNEMARIE et al. "Ring cleavage of O,N-heterocycles. 9. Synthesis and reactivity of 4H-1,3,4-oxa- diazolo(2,3-c)-as-traizines" Seite 505, Spalte 1, Zu- sammenfassung-Nr. 131 552t
- CHEMICAL ABSTRACTS, Band 102, Nr. 25, 24. Juni 1985, Columbus, Ohio, USA SHOWA DENKO K.K. "Oxadiazolinones" Seite 595, Spalte 2, Zusammenfassung-Nr. 220 884f
- CHEMICAL ABSTRACTS, Band 91, Nr. 25, 17. Dezember 1979, Columbus, Ohio, USA TAKANO, TADAYOSHI "7-Aminocephalosporanic acid oxadiazoleacetamide derivatives" Seite 687, Spalte 2, Zusammenfassung-Nr. 211 409g
- CHEMICAL ABSTRACTS, Band 100, Nr. 9, 27. Februar 1984, Columbus, Ohio, USA MUSSER, JOHN H. et al. "Synthesis of 2-(2,3-dihydro- 2-oxo-1,3,4-oxadiazol-5- yl) benzo heterocycles. A novel series of orally active antiallergic agents" Seite 594, Spalte 1, Zusammenfassung-Nr. 68 231q
- CHEMICAL ABSTRACTS, Band 81, Nr. 25, 23. Dezember 1974, Columbus, Ohio, USA AKERBLOM, EVA B. "Nitrofurans with high renal excretion" Seite 19, Spalte 2, Zusammenfassung-Nr. 163 330u

## Beschreibung

Die vorliegende Erfindung betrifft ein neuartiges Verfahren zur Herstellung von 4-Amino-3-oxo-2,3,4,5-tetrahydro-1,2,4-triazinen.

Gegenstand der Erfindung ist ein Verfahren zur Herstellung einer Verbindung der Formel I 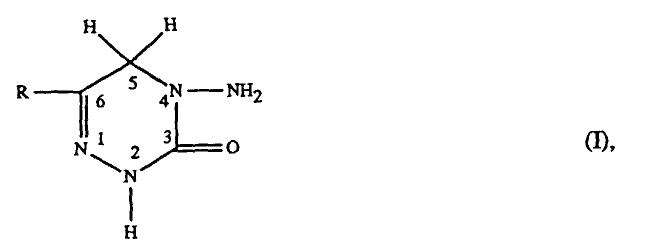 worin R Wasserstoff, C₁-C₆-Alkyl, C₃-C₆-Cycloalkyl, mit 1 oder mehreren Halogenatomen oder 1 bis 3 Resten aus der Gruppe C₁-C₃-Alkoxy, C₁-C₃-Alkylthio oder Phenyl substituiertes C₁-C₄-Alkyl, Phenyl oder mit 1 bis 3 Resten aus der Gruppe Halogen, Methyl, Aethyl, Methoxy, Methylthio oder Nitro substituiertes Phenyl bedeutet, welches dadurch gekennzeichnet, ist, dass man eine Verbindung der Formel II 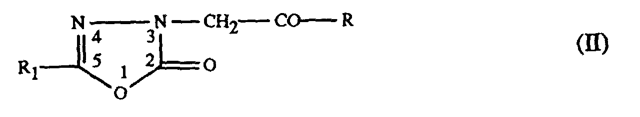 worin R₁ Wasserstoff, C₁-C₄-Alkyl, C₃-C₆-Cycloalkyl, mit 1 bis 9 Chloratomen substituiertes C₁-C₄-Alkyl, C₁-C₃-Alkoxy, C₁-C₃-Alkylthio, C₁-C₃-Alkylsulfinyl, C₁-C₃-Alkylsulfonyl, Phenyl, mit 1 bis 3 Resten aus der Gruppe Halogen, Methyl, Aethyl, Methoxy, Methylthio oder Nitro substituiertes Phenyl oder Pyridyl bedeutet, und
R die oben angegebenen Bedeutungen besitzt; mit Hydrazinhydrat umsetzt und die erhaltene Verbindung der Formel III, 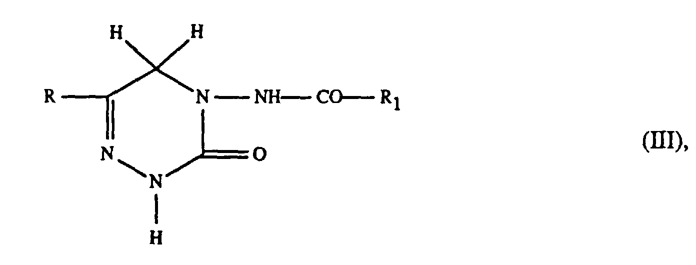 vorzugsweise sauer, verseift.

Das vorliegende Verfahren dient bevorzugt zur Herstellung von Verbindungen der Formel I, worin R für Methyl, Aethyl, Isopropyl, tert.Butyl oder Cyclopropyl steht. Das Verfahren wird vorzugsweise mit Verbindungen der Formel II als Ausgangsprodukte durchgeführt, worin R₁ C₁-C₄-Alkyl bedeutet.

Die erfindungsgemäss hergestellten Aminotriazinderivate der Formel I können als Zwischenprodukte zur Herstellung von 4-[(Pyrid-3-yl)-methylenamino]- bzw. 4-[(Pyrid-3-yl)-methylamino]-3-oxo-2,3,4,5-tetrahydro-1,2,4-triazinen, die sich durch eine ausgeprägte insektizide und akarizide Wirkung auszeichnen, verwendet werden. Als solche pestiziden Wirkstoffe seien z.B. 4-[(Pyrid-3-yl)-methylenamino]-3-oxo-6-methyl-2,3,4,5-tetrahydro-1,2,4-triazin, 4-[(Pyrid-3-yl)-methylenamino]-3-oxo-6-cyclopropyl-2,3,4,5-tetrahydro-1,2,4-triazin, 4-[(Pyrid-3-yl)-methylamino]-3-oxo-6-isopropyl-2,3,4,5-tetrahydro-1,2,4-triazin und 4-[(Pyrid-3-yl)-methylamino]-3-oxo-6-tert.butyl-2,3,4,5-tetrahydro-1,2,4-triazin genannt. Derartige pestizide Wirkstoffe, ihre Herstellung und Verwendung werden in der EP-Patentanmeldung 314,615 beschrieben.

Das erfindungsgemässe Verfahren kann durch das folgende Reaktionsschema wiedergegeben werden; wobei die Reste R und R₁ die oben angegebenen Bedeutungen haben:

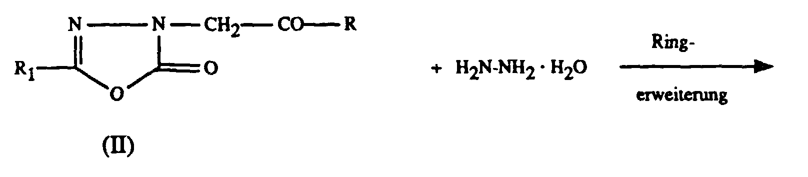

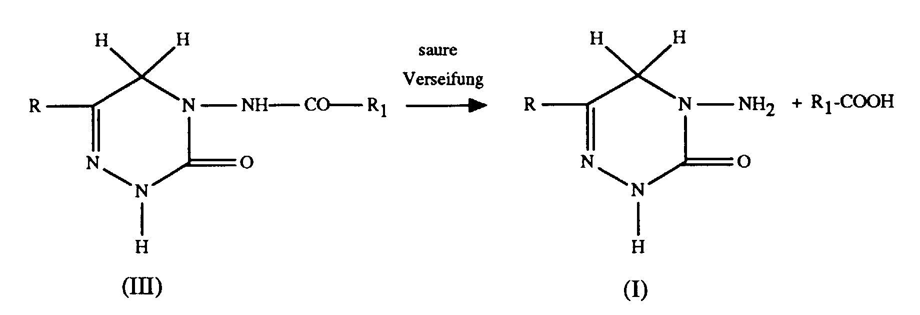

Der erste Schritt (Ringerweiterung) des erfindungsgemässen Verfahrens zur Herstellung der Verbindungen der Formel I wird üblicherweise unter normalem Druck und vorzugsweise in einem Lösungsmittel durchgeführt. Die Temperatur beträgt dabei zwischen +15 und 120°C, vorzugsweise zwischen +20 und 100°C. Geeignete Lösungsmittel sind z.B. Wasser, Nitrile wie Acetonitril, Alkohole, Dioxan oder Tetrahydrofuran. Die nachfolgende Verseifung der Acylaminoverbindungen der Formel III zu den freien Aminoverbindungen der Formel I wird vorzugsweise mit anorganischen Säuren, wie 1n Salzsäure bis konz. Salzsäure oder 1n- bis 10n-Schwefelsäure, bei Temperaturen von 0-120°C, speziell +20 bis 100°C, in wässrigem Medium oder in organischen Lösungsmitteln, wie Alkoholen, Dioxan, Tetrahydrofuran, oder Nitrilen durchgeführt.

Die erfindungsgemäss als Ausgangsprodukte verwendeten 1,3,4-Oxadiazolin-2-on-3-yl-ketone der Formel II sind neu. Sie können analog bekannter Arbeitsweisen z.B. wie folgt hergestellt werden (vgl. z.B. EP-Patentanmeldung No. 314,615):

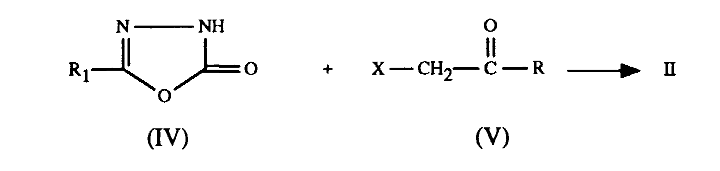

In den obigen Formeln IV und V haben R und R₁ die vorstehend angegebenen Bedeutungen, und X steht für ein Halogenatom, vorzugsweise Chlor oder Brom. Das obige Verfahren zur Herstellung der Oxadiazolinon-ketone der Formel II wird im allgemeinen bei normalem Druck in Gegenwart einer Base und in einem Lösungsmittel durchgeführt. Die Temperatur beträgt dabei zwischen 0 und +150°C, vorzugsweise zwischen +20 und 100°C. Als Basen eignen sich organische und anorganische Basen, wie z.B. Trimethylamin, Alkoholate, Natriumhydroxid oder Natriumhydrid. Als Lösungsmittel eignen sich u.a. Alkohole, halogenierte Kohlenwasserstoffe, wie z.B. Chloroform, Nitrile, wie z.B. Acetonitril, Tetrahydrofuran, Dioxan, Dimethylsulfoxid, Dimethylformamid oder auch Wasser.

Die Oxadiazolinone der Formel IV [vgl. EP-Patentanmeldung No. 321,833; J. Pharm. Soc. Japan 76, 1300-1303 (1956); B. 82, 121-123 (1949)] und ihre Herstellung, sowie die Halogenketone der Formel V sind grösstenteils bekannt.

Aus Liebigs Ann. Chem. 749, 125 ff. (1971) ist es bekannt, dass man ausgehend von 2-Amino-5-methyl-3-phenacyl-1,3,4-oxadiazoliumbromid durch Umsetzung mit Hydrazinhydrat zu 4-Amino-6-phenyl-3-oxo-2,3,4,5-tetrahydro-1,2,4-triazinen gelangt. Der Hauptnachteil dieses Verfahrens besteht darin, dass es auf die Herstellung von 1,2,4-Triazinringen mit Phenylsubstitution in der 6-Stellung begrenzt ist; dieses Verfahren ist ausserdem mehrstufig und seine Ausbeute niedrig. Es ist weiterhin aus der EP-Patentanmeldung Nr. 314,615 bekannt, in 6-Stellung substituierte 4-Amino-3-oxo-2,3,4,5-tetrahydro-1,2,4-triazine herzustellen, und zwar durch einstufige Umsetzung von entsprechenden 5-Trifluormethyl-1,3,4-oxadiazolin-2-on-3-yl-ketonen mit überschüssigem Hydrazin: 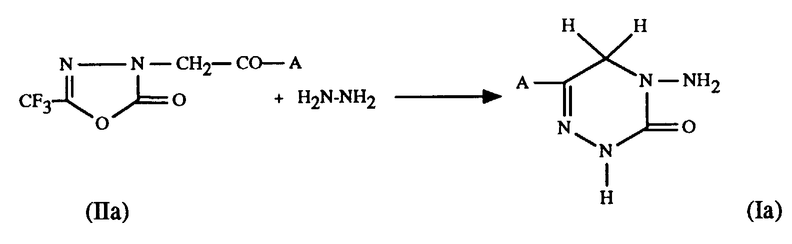 wobei A ein gegebenenfalls substituierter Alkyl- oder Aryl-Substituent sein kann. Nachteilig an diesem Verfahren ist primär der hohe Preis des zur Herstellung des Trifluoracethydrazids benötigten Trifluoressigsäureäthylesters, die Instabilität dieses Trifluoracethydrazids bei Raumtemperatur und die nicht sehr hohe Ausbeute bei der Umsetzung des Trifluoracethydrazids mit Phosgen in Wasser (vgl. Helv. Chim. Acta 1986, 333) zwecks Herstellung des 5-Trifluormethyl-1,3,4-oxadiazol-2(3H)-ons, -2(3H)-ons, von dem aus man zu den 5-Trifluormethyl-1,3,4-oxadiazolin-2-on-3-yl-ketonen der obigen Formel IIa (Ausgangsverbindung) gelangt. Ferner entstehen bei der Umsetzung gemäss EP-Patentanmeldung Nr. 314,615 als Nebenprodukt zwangsläufig toxische Trifluoressigsäure-Derivate, die aus ökologischen Gründen problematisch sind und einen erheblichen Entsorgungsaufwand erfordern.

Demgegenüber wurde nun im Rahmen der vorliegenden Erfindung überraschenderweise festgestellt, dass zur Herstellung der 4-Amino-3-oxo-2,3,4,5-tetrahydro-1,2,4-triazine der Formel I durch Ringerweiterung das Vorliegen einer 5-CF₃-Gruppe in den Ausgangsverbindungen der Formel II nicht notwendig ist. Die vorliegenden Ausgangsverbindungen der Formel II, die in 5-Stellung statt der erwähnten CF₃-Gruppe einen der angegebenen Reste R₁ aufweisen, reagieren ohne weiteres mit Hydrazinhydrat unter Bildung der Acylaminoverbindungen der Formel III, aus denen allerdings nachträglich der Rest -CO-R₁ durch saure Verseifung abgespalten werden muss, um die Verbindungen der Formel I zu erhalten. Mit dem erfindungsgemässen Verfahren werden die Nachteile der bisherigen Arbeitsweisen ausgeschaltet, da bei dem erfindungsgemässen Verfahren preiswerte und leicht zugängliche Ausgangsverbindungen verwendet werden können, hohe Ausbeuten erzielt werden und anstatt toxischer Trifluoressigsäurederivate als Nebenprodukte ökolologisch unbedenkliche Carbonsäurederivate, wie z.B. Essigsäure, entstehen.

Die Verbindungen der Formel 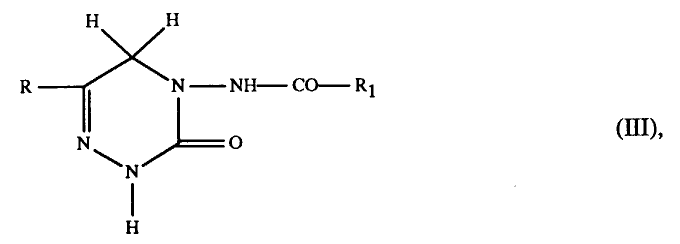 worin R und R₁ die in Anspruch 1 angegebenen Bedeutungen haben, mit der Massgabe, dass in den Verbindungen der Formel III, worin R Phenyl ist, R₁ von Methyl, n-Butyl und Phenyl verschieden ist, sind neu und bilden ebenfalls einen Gegenstand der vorliegenden Erfindung.

### Beispiel 1: Herstellung der Ausgangsverbindung

### 2,3-Dihydro-5-methyl-2-oxo-1,3,4-oxadiazol-3-aceton

Man gibt zu einer Lösung von 2,3 g Natrium in 100 ml Methanol 10 g 2,3-Dihydro-5-methyl-2-oxo-1,3,4-oxadiazol (auf übliche Wesie hergestellt aus Acethydrazid und Phosgen), verrührt diese Mischung während kurzer Zeit und entfernt anschliessend das Lösungsmittel im Vakuum bei 60°C Badtemperatur. Das so entstandene Natriumsalz wird portionenweise zu einer Lösung von 9,2 g Chloraceton und 0,2 g Tetrabutylammoniumbromid in 50 ml Chloroform eingetragen und die Reaktionsmischung während 4 Stunden bei 65°C verrührt. Nach Abfiltrieren der Salze entfernt man das Lösungsmittel im Vakuum bei 50°C Badtemperatur. Der hinterbleibende Rückstand wird aus t-Butylmethyläther umkristallisiert und ergibt die Titelverbindung vom Schmelzpunkt 55-57°C.

In entsprechender Weise wie oben angegeben werden auch die folgenden Verbindungen der Formel II hergestellt:

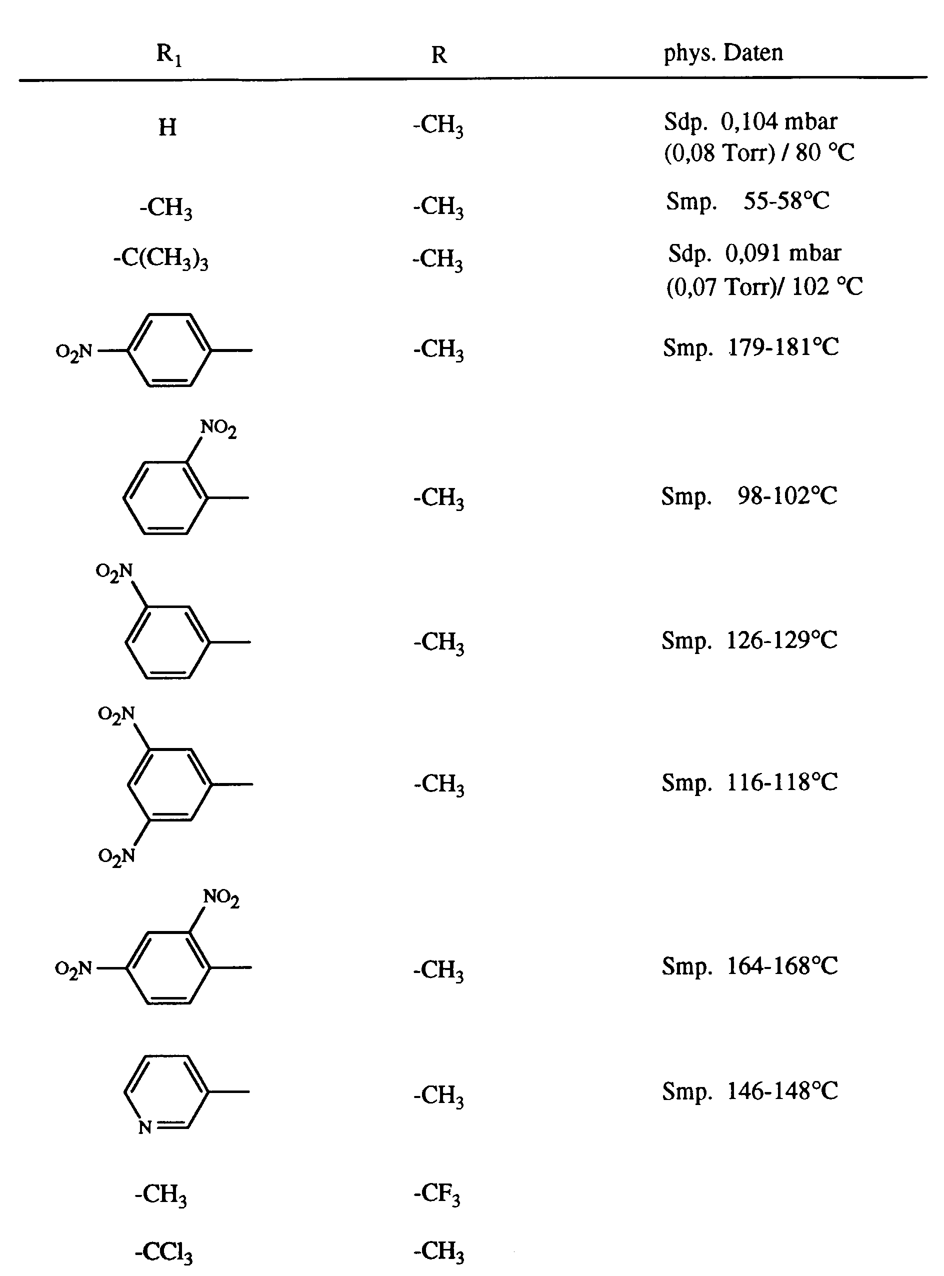

### Beispiel 2:

### a) Herstellung von 4-Acetylamino-6-methyl-3-oxo-2,3,4,5-tetrahydro-1,2,4-triazin (Ringerweiterung):

3,12 g des gemäss Beispiel 1 hergestellten 2,3-Dihydro-5-methyl-2-oxo-1,3,4-oxadiazol-3-acetons werden in 40 ml Alkohol zusammen mit 2 g Hydrazinhydrat während 16 Stunden bei 35°C Badtemperatur verrührt. Nach dem Abdampfen des Lösungsmittels und des Hydrazinüberschusses im Vakuum erhält man nach Umkristallisation aus Isopropanol die Titelverbindung vom Schmelzpunkt 197-199°C.

In entsprechender Weise wie oben angegeben werden auch die folgenden Verbindungen der Formel III hergestellt:

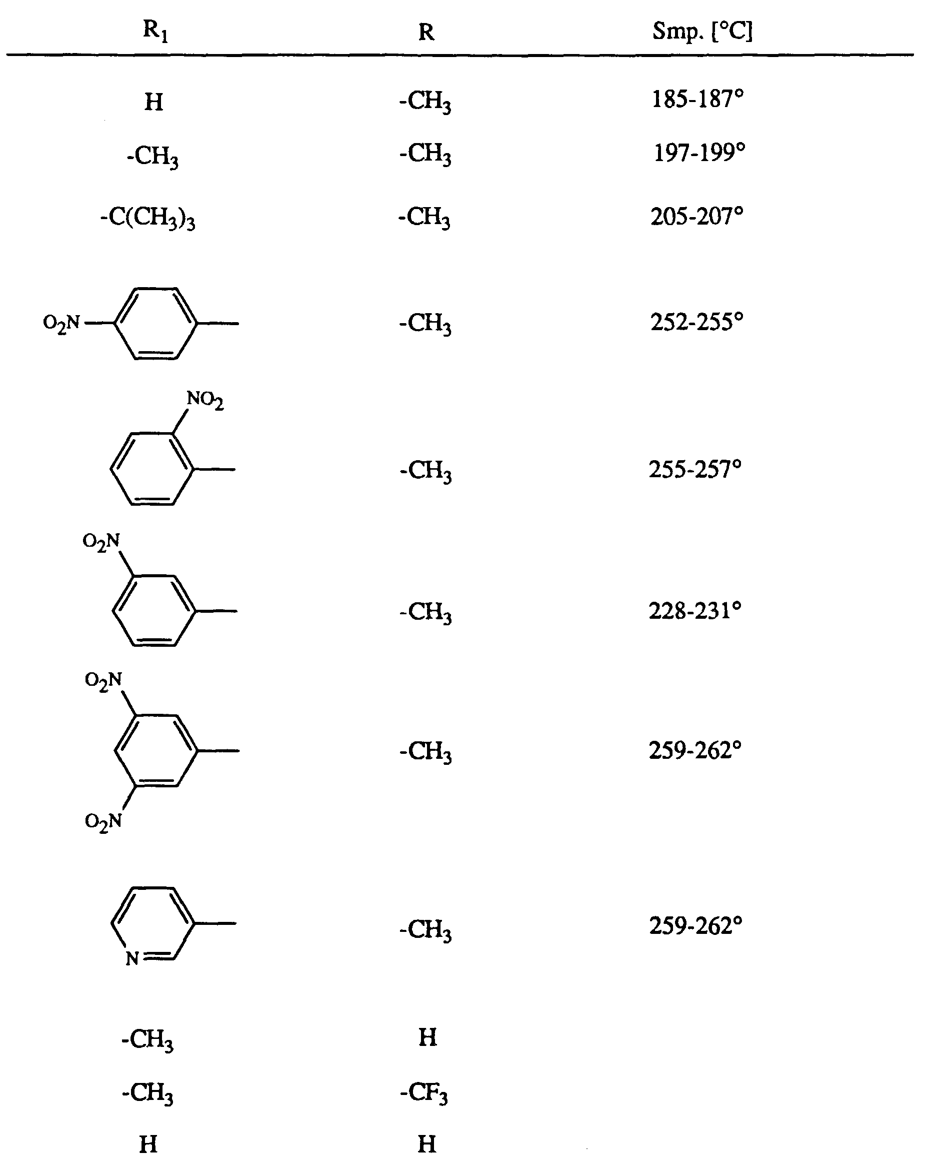

### b) Herstellung von 4-Amino-6-methyl-3-oxo-2,3,4,5-tetrahydro-1,2,4-triazin (saure Verseifung):

1,7 g des wie vorstehend gemäss a) hergestellten 4-Acetylamino-6-methyl-3-oxo-2,3,4,5-tetrahydro-1,2,4-triazins werden während 5 Stunden in 10 ml 2-n Salzsäure bei 80°C verrührt. Nach dem Erkalten der Lösung gibt man 1,7 g Natriumacetat hinzu und dampft die Lösung am Rotationsverdampfer bei 60°C Badtemperatur ein. Der gebildete Rückstand wird mit Äthanol verrührt und durch Filtration von Salzausfällungen befreit. Nach Einengen der erhaltenen Lösung auf ein kleines Volumen wird dieses zur Kristallisation gebracht. Man erhält die Titelverbindung in Form farbloser Kristalle vom Schmelzpunkt 116-119°C.

In entsprechender Weise wie oben angegeben werden auch die folgenden Verbindungen der Formel I hergestellt:

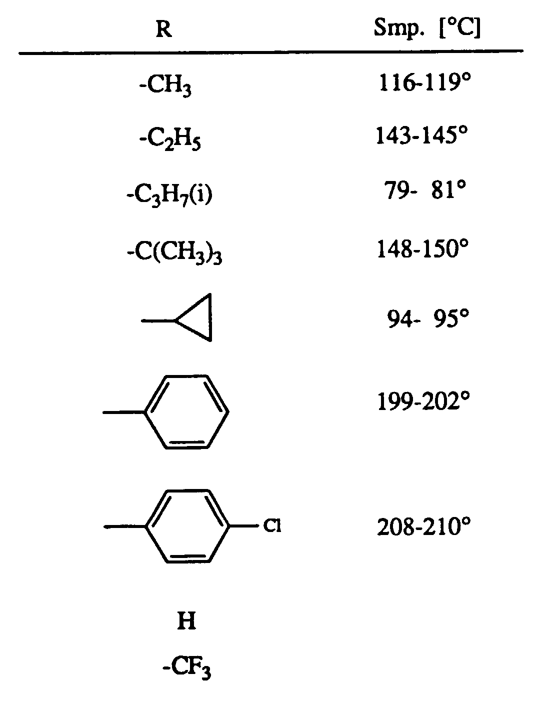

## Patentansprüche (Patentansprüche für folgende(n) Vertragsstaat(en): BE, CH, DE, DK, FR, GB, GR, IT, LI, LU, NL)

1. Verfahren zur Herstellung einer Verbindung der Formel I 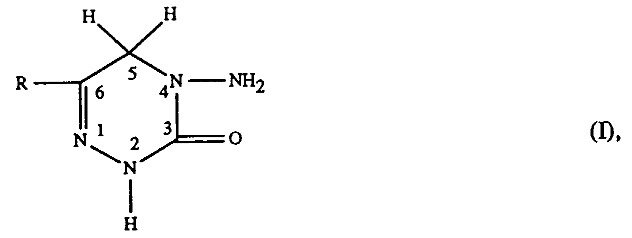 worin
R Wasserstoff, C₁-C₆-Alkyl, C₃-C₆-Cycloalkyl, mit 1 oder mehreren Halogenatomen oder 1 bis 3 Resten aus der Gruppe C₁-C₃-Alkoxy, C₁-C₃-Alkylthio oder Phenyl substituiertes C₁-C₄-Alkyl, Phenyl oder mit 1 bis 3 Resten aus der Gruppe Halogen, Methyl, Aethyl, Methoxy, Methylthio oder Nitro substituiertes Phenyl bedeutet, dadurch gekennzeichnet, dass man eine Verbindung der Formel II 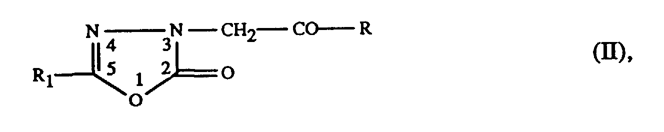 worin R₁ Wasserstoff, C₁-C₄-Alkyl, C₃-C₆-Cycloalkyl, mit 1 bis 9 Chloratomen substituiertes C₁-C₄-Alkyl, C₁-C₃-Alkoxy, C₁-C₃-Alkylthio, C₁-C₃-Alkylsulfinyl, C₁-C₃-Alkylsulfonyl, Phenyl, mit 1 bis 3 Resten aus der Gruppe Halogen, Methyl, Aethyl, Methoxy, Methylthio oder Nitro substituiertes Phenyl oder Pyridyl bedeutet; und
R die oben angegebenen Bedeutungen besitzt; mit Hydrazinhydrat umsetzt und die erhaltene Verbindung der Formel III 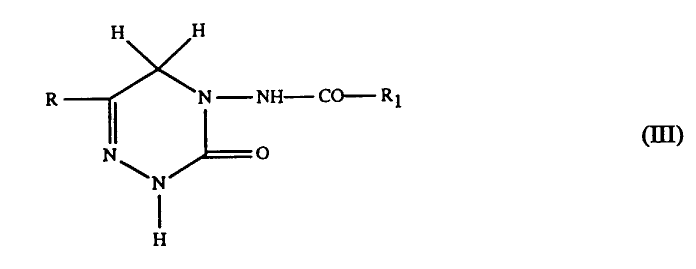 verseift.

2. Verfahren gemäss Anspruch 1, dadurch gekennzeichnet, dass man die Verbindung der Formel III sauer verseift.

3. Verfahren gemäss Anspruch 1 oder 2, dadurch gekennzeichnet, dass man die Verbindung der Formel III mit einer anorganischen Säure verseift.

4. Verfahren gemäss Anspruch 1 bis 3 zur Herstellung einer Verbindung der Formel I, worin R Methyl, Aethyl, Isopropyl, tert.Butyl oder Cyclopropyl bedeutet.

5. Verfahren gemäss Anspruch 1 bis 4, dadurch gekennzeichnet, dass man eine Verbindung der Formel II umsetzt, worin R₁ C₁-C₄-Alkyl bedeutet.

6. Verbindung der Formel 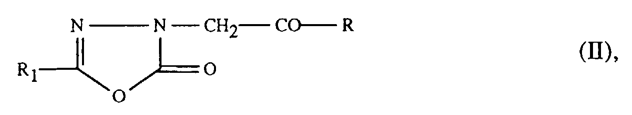 worin R und R₁ die in Anspruch 1 angegebenen Bedeutungen haben.

7. Verbindung der Formel 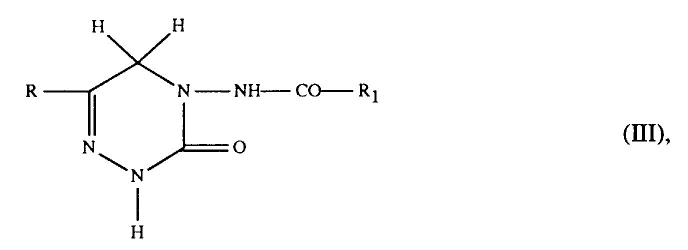 worin R und R₁ die in Anspruch 1 angegebenen Bedeutungen haben, mit der Massgabe, dass in den Verbindungen der Formel III, worin R Phenyl ist, R₁ von Methyl, n-Butyl und Phenyl verschieden ist.

## Patentansprüche (Patentansprüche für folgende(n) Vertragsstaat(en): ES)

1. Verfahren zur Herstellung einer Verbindung der Formel I 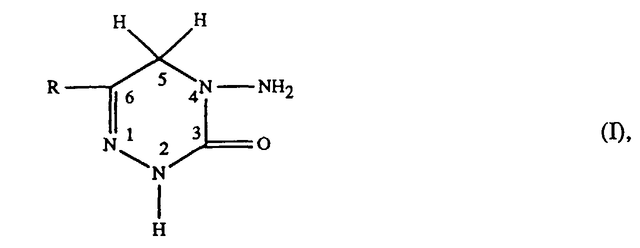 worin
R Wasserstoff, C₁-C₆-Alkyl, C₃-C₆-Cycloalkyl, mit 1 oder mehreren Halogenatomen oder 1 bis 3 Resten aus der Gruppe C₁-C₃-Alkoxy, C₁-C₃-Alkylthio oder Phenyl substituiertes C₁-C₄-Alkyl, Phenyl oder mit 1 bis 3 Resten aus der Gruppe Halogen, Methyl, Aethyl, Methoxy, Methylthio oder Nitro substituiertes Phenyl bedeutet, dadurch gekennzeichnet, dass man eine Verbindung der Formel II 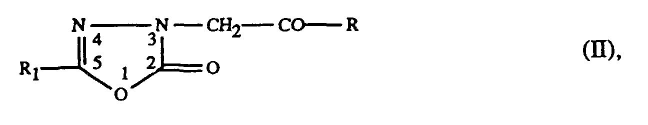 worin R₁ Wasserstoff, C₁-C₄-Alkyl, C₃-C₆-Cycloalkyl, mit 1 bis 9 Chloratomen substituiertes C₁-C₄-Alkyl, C₁-C₃-Alkoxy, C₁-C₃-Alkylthio, C₁-C₃-Alkylsulfinyl, C₁-C₃-Alkylsulfonyl, Phenyl, mit 1 bis 3 Resten aus der Gruppe Halogen, Methyl, Aethyl, Methoxy, Methylthio oder Nitro substituiertes Phenyl oder Pyridyl bedeutet; und
R die oben angegebenen Bedeutungen besitzt; mit Hydrazinhydrat umsetzt und die erhaltene Verbindung der Formel III 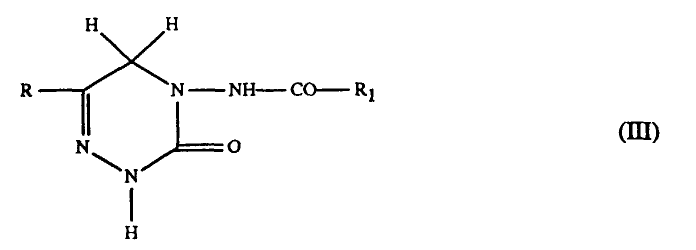 verseift.

2. Verfahren gemäss Anspruch 1, dadurch gekennzeichnet, dass man die Verbindung der Formel III sauer verseift.

3. Verfahren gemäss Anspruch 1 oder 2, dadurch gekennzeichnet, dass man die Verbindung der Formel III mit einer anorganischen Säure verseift.

4. Verfahren gemäss Anspruch 1 bis 3 zur Herstellung einer Verbindung der Formel I, worin R Methyl, Aethyl, Isopropyl, tert.Butyl oder Cyclopropyl bedeutet.

5. Verfahren gemäss Anspruch 1 bis 4, dadurch gekennzeichnet, dass man eine Verbindung der Formel II umsetzt, worin R₁ C₁-C₄-Alkyl bedeutet.

6. Verfahren zur Herstellung einer Verbindung der Formel 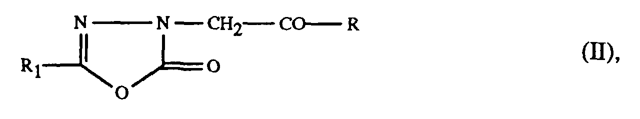 worin R und R₁ die in Anspruch 1 angegebenen Bedeutungen haben, dadurch gekennzeichnet, dass man eine Verbindung der Formel 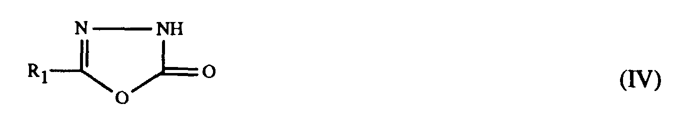 mit einer Verbindung der Formel 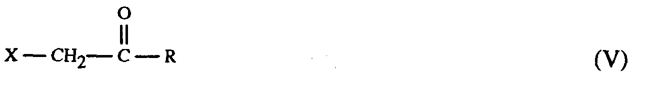 umsetzt, wobei R und R₁ in den Verbindungen der Formeln IV bzw. V die in Anspruch 1 angegebenen Bedeutungen haben, und X für ein Halogenatom steht.

7. Verfahren zur Herstellung einer Verbindung der Formel 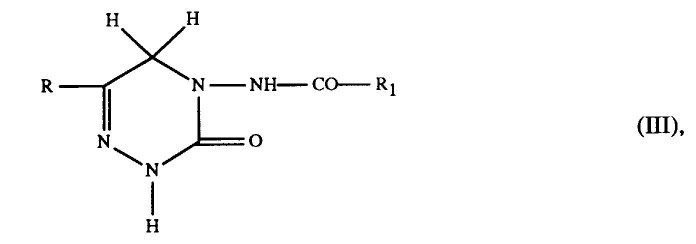 worin R und R₁ die in Anspruch 1 angegebenen Bedeutungen haben, dadurch gekennzeichnet, dass man eine Verbindung der Formel II 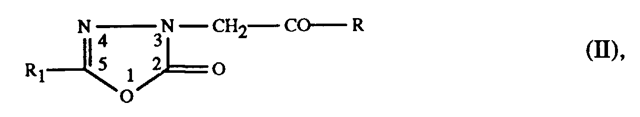 worin R₁ und R₁ die in Anspruch 1 angegebenen Bedeutungen besitzt, mit Hydrazinhydrat umsetzt.

## Claims (Claims for the following Contracting State(s): BE, CH, DE, DK, FR, GB, GR, IT, LI, LU, NL)

1. A process for the preparation of a compound of formula I 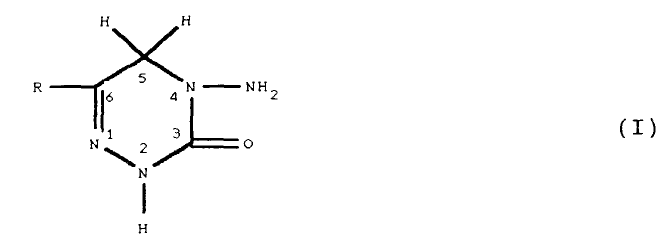 wherein
R is hydrogen, C₁-C₆alkyl, C₃-C₆cycloalkyl, C₁-C₄alkyl substituted by one or more halogen atoms or by from 1 to 3 radicals from the group C₁-C₃alkoxy, C₁-C₃alkylthio and phenyl, phenyl or phenyl substituted by from 1 to 3 radicals from the group halogen, methyl, ethyl, methoxy, methylthio and nitro, which process comprises reacting with hydrazine hydrate a compound of formula II 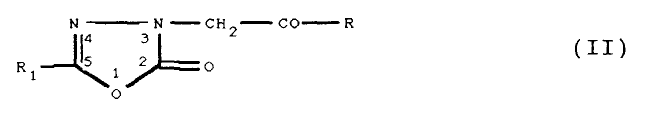 wherein R₁ is hydrogen, C₁-C₄alkyl, C₃-C₆cycloalkyl, C₁-C₄-alkyl substituted by from 1 to 9 chlorine atoms, C₁-C₃alkoxy, C₁-C₃alkylthio, C₁-C₃alkylsulfinyl, C₁-C₃alkylsulfonyl, phenyl, phenyl substituted by from 1 to 3 radicals from the group halogen, methyl, ethyl, methoxy, methylthio and nitro, or pyridyl; and
R is as defined above; and subjecting the resulting compound of formula III 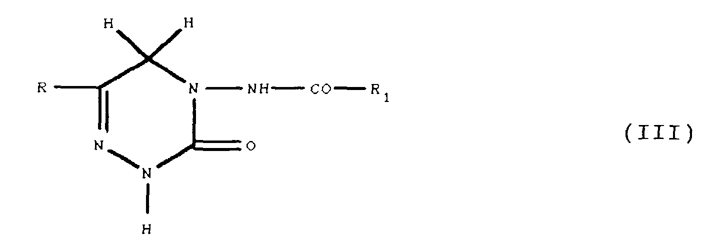 to hydrolysis.

2. A process according to claim 1, wherein the compound of formula III is subjected to acid hydrolysis.

3. A process according to claim 1 or 2, wherein the compound of formula III is subjected to hydrolysis with an inorganic acid.

4. A process according to any one of claims 1 to 3 for the preparation of a compound of formula I wherein R is methyl, ethyl, isopropyl, tert-butyl or cyclopropyl.

5. A process according to any one of claims 1 to 4, wherein a compound of formula II wherein R₁ is C₁-C₄alkyl is reacted.

6. A compound of the formula 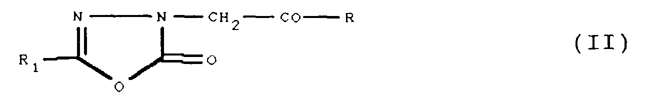 wherein R and R₁ are as defined in claim 1.

7. A compound of the formula 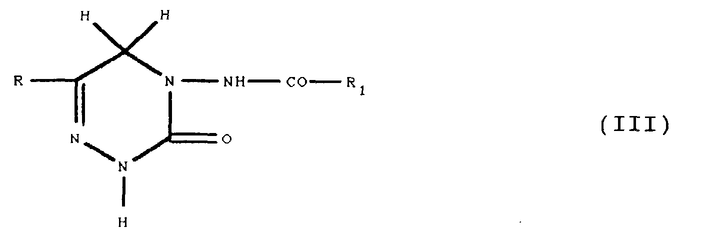 wherein R and R₁ are as defined in claim 1, with the proviso that in compounds of formula III wherein R is phenyl, R₁ is other than methyl, n-butyl and phenyl.

## Claims (Claims for the following Contracting State(s): ES)

1. A process for the preparation of a compound of formula I 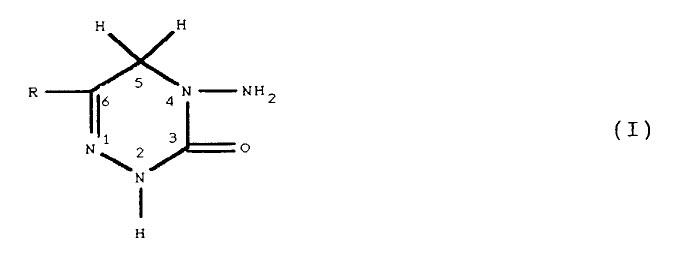 wherein
R is hydrogen, C₁-C₆alkyl, C₃-C₆cycloalkyl, C₁-C₄alkyl substituted by one or more halogen atoms or by from 1 to 3 radicals from the group C₁-C₃alkoxy, C₁-C₃alkylthio and phenyl, phenyl or phenyl substituted by from 1 to 3 radicals from the group halogen, methyl, ethyl, methoxy, methylthio and nitro, which process comprises reacting with hydrazine hydrate a compound of formula II 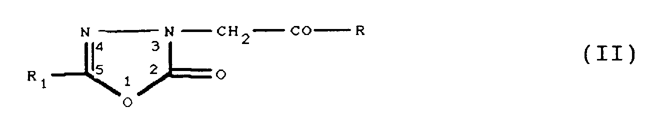 wherein R₁ is hydrogen, C₁-C₄alkyl, C₃-C₆cycloalkyl, C₁-C₄alkyl substituted by from 1 to 9 chlorine atoms, C₁-C₃alkoxy, C₁-C₃alkylthio, C₁-C₃alkylsulfinyl, C₁-C₃alkylsulfonyl, phenyl, phenyl substituted by from 1 to 3 radicals from the group halogen, methyl, ethyl, methoxy, methylthio and nitro, or pyridyl; and
R is as defined above; and subjecting the resulting compound of formula III 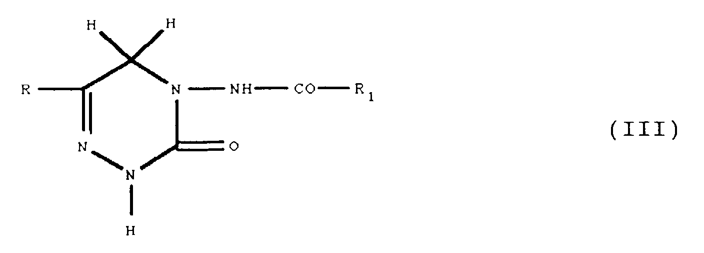 to hydrolysis.

2. A process according to claim 1, wherein the compound of formula III is subjected to acid hydrolysis.

3. A process according to claim 1 or 2, wherein the compound of formula III is subjected to hydrolysis with an inorganic acid.

4. A process according to any one of claims 1 to 3 for the preparation of a compound of formula I wherein R is methyl, ethyl, isopropyl, tert-butyl or cyclopropyl.

5. A process according to any one of claims 1 to 4, wherein a compound of formula II wherein R₁ is C₁-C₄alkyl is reacted.

6. A process for the preparation of a compound of the formula 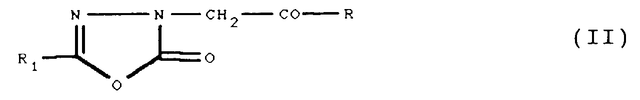 wherein R and R₁ are as defined in claim 1, which comprises reacting a compound of the formula 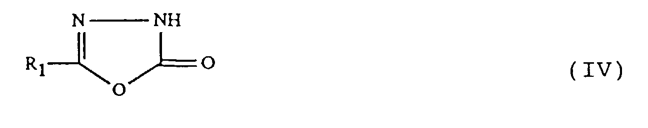 with a compound of the formula 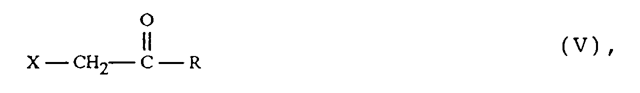 R and R₁ in the compounds of formulae IV and V being as defined in claim 1, and X being a halogen atom.

7. A process for the preparation of a compound of the formula 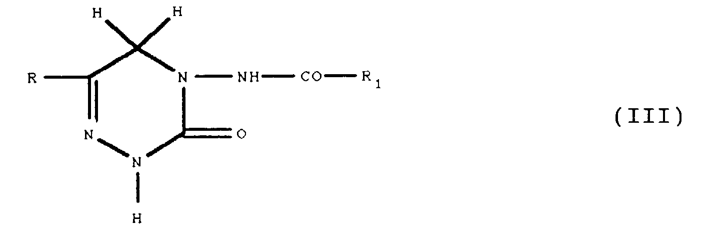 wherein R and R₁ are as defined in claim 1, which comprises reacting with hydrazine hydrate a compound of formula II 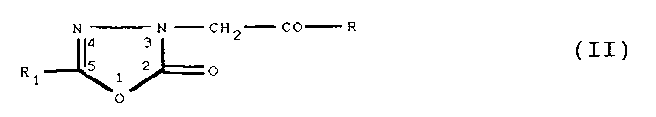 wherein R₁ and R₁ are as defined in claim 1.

## Revendications (Revendications pour l'(les) Etat(s) contractant(s) suivant(s): BE, CH, DE, DK, FR, GB, GR, IT, LI, LU, NL)

1. Procédé de préparation d'un composé de formule I 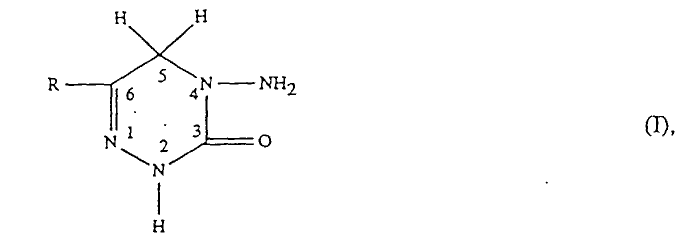 dans laquelle R représente un hydrogène, un alkyle en C₁₋₆, un cycloalkyle en C₃₋₆, un alkyle en C₁₋₄ substitué par un ou plusieurs atomes d'halogène ou 1 à 3 radicaux du groupe alcoxy en C₁₋₃, alkylthio en C₁₋₃ ou phényle, un phényle ou un phényle substitué par de 1 à 3 radicaux du groupe halogène, méthyle, éthyle, méthoxy, méthylthio ou nitro, qui se caractérise en ce qu'on fait réagir avec de l'hydrazine hydratée un composé de formule II 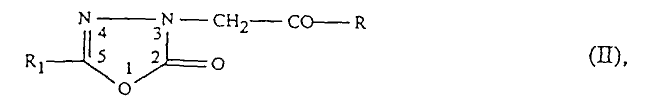 dans laquelle R₁ représente un hydrogène, un alkyle en C₁₋₄, un cycloalkyle en C₃₋₆, un alkyle en C₁₋₄ substitué par de 1 à 9 atomes de chlore, un alcoxy en C₁₋₃, un alkylthio en C₁₋₃, un alkyle en C₁₋₃ sulfinyle, un alkyle en C₁₋₃ sulfonyle, un phényle, un phényle ou un pyridyle substitué par de 1 à 3 radicaux du groupe halogène, méthyle, éthyle, méthoxy, méthylthio ou nitro, et R a les significations indiquées ci-dessus ; et ce qu'on effectue une saponification du composé obtenu de formule III

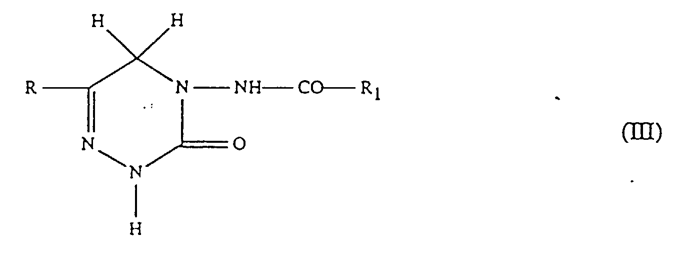

2. Procédé selon la revendication 1, caractérisé en ce qu'on effectue une saponification acide du composé de formule III.

3. Procédé selon la revendication 1 ou 2, caractérisé en ce qu'on saponifie le composé de formule III avec un acide inorganique.

4. Procédé selon les revendications 1 à 3 de préparation d'un composé de formule I, dans laquelle R représente un méthyle, un éthyle, un isopropyle, un butyle tertiaire ou un cyclopropyle.

5. Procédé selon l'une des revendications 1 à 4, caractérisé en ce qu'on fait réagir un composé de formule II dans lequel R₁ représente un alkyle en C₁₋₄.

6. Composé de formule 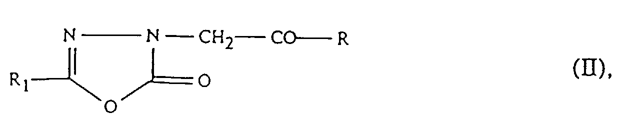 dans laquelle R et R₁ ont les significations indiquées dans la revendication 1.

7. Composé de formule 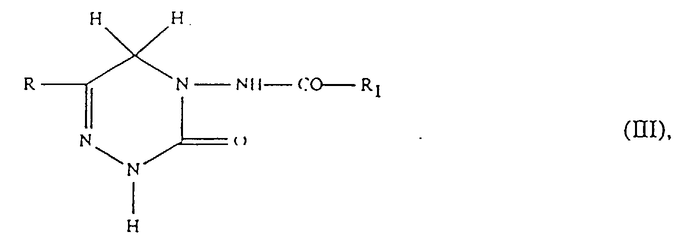 dans laquelle R et R₁ ont les significations indiquées dans la revendication 1, sous réserve que dans les composés de formule III dans laquelle R₁ est un phényle, R₁ est différent d'un méthyle, d'un n-butyle et d'un phényle.

## Revendications (Revendications pour l'(les) Etat(s) contractant(s) suivant(s): ES)

1. Procédé de préparation d'un composé de formule I 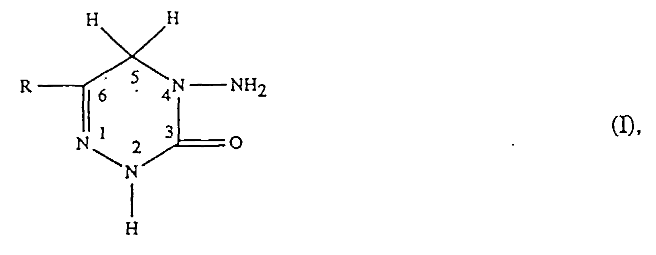 dans laquelle R représente un hydrogène, un alkyle en C₁₋₆, un cycloalkyle en C₃₋₆, un alkyle en C₁₋₄ substitué par un ou plusieurs atomes d'halogène ou 1 à 3 radicaux du groupe alcoxy en C₁₋₃, alkylthio en C₁₋₃ ou phényle, un phényle ou un phényle substitué par de 1 à 3 radicaux du groupe halogène, méthyle, éthyle, méthoxy, méthylthio ou nitro, qui se caractérise en ce qu'on fait réagir avec de l'hydrazine hydratée un composé de formule II 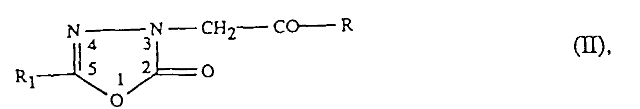 dans laquelle R₁ représente un hydrogène, un alkyle en C₁₋₄, un cycloalkyle en C₃₋₆, un alkyle en C₁₋₄ substitué par de 1 à 9 atomes de chlore, un alcoxy en C₁₋₃, un alkylthio en C₁₋₃, un alkyle en C₁₋₃ sulfinyle, un alkyle en C₁₋₃ sulfonyle, un phényle, un phényle ou un pyridyle substitué par de 1 à 3 radicaux du groupe halogène, méthyle, éthyle, méthoxy, méthylthio ou nitro, et R a les significations indiquées ci-dessus ; et ce qu'on effectue une saponification du composé obtenu de formule III

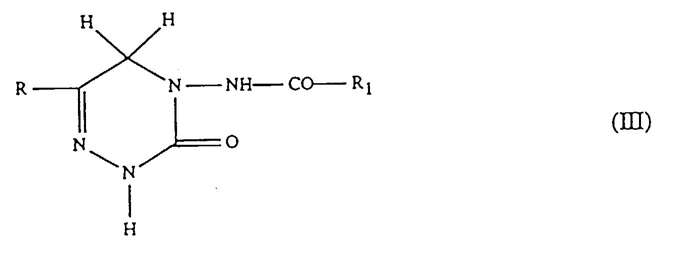

2. Procédé selon la revendication 1, caractérisé en ce qu'on effectue une saponification acide du composé de formule III.

3. Procédé selon la revendication 1 ou 2, caractérisé en ce qu'on saponifie le composé de formule III avec un acide inorganique.

4. Procédé selon les revendications 1 à 3 de préparation d'un composé de formule I, dans laquelle R représente un méthyle, un éthyle, un isopropyle, un butyle tertiaire ou un cyclopropyle.

5. Procédé selon l'une des revendications 1 à 4, caractérisé en ce qu'on fait réagir un composé de formule II dans lequel R1 représente un alkyle en C₁₋₄.

6. Procédé de préparation d'un composé de formule 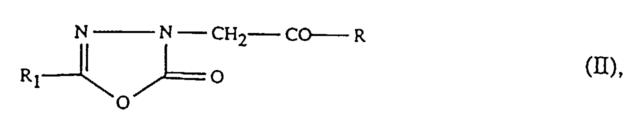 dans laquelle R et R₁ ont les significations indiquées dans la revendication 1, caractérisé en ce qu'on fait réagir un composé de formule 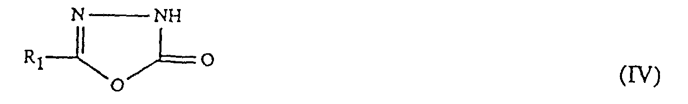 avec un composé de formule 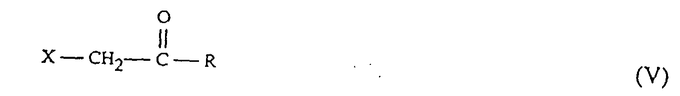 dans lesquels R et R₁, dans les composés de formule IV ou V, ont les significations indiquées dans la revendication 1, et X représente un atome d'halogène.

7. Procédé de préparation d'un composé de formule 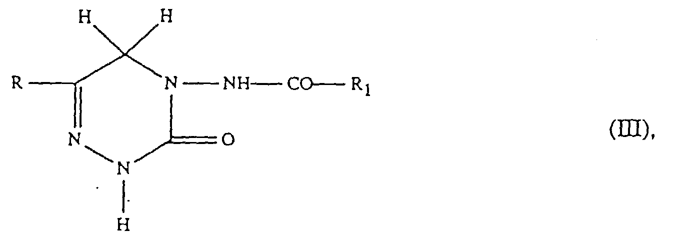 dans laquelle R et R₁ ont les significations indiquées dans la revendication 1, caractérisé en ce qu'on fait réagir avec de l'hydrazine hydratée un composé de formule II 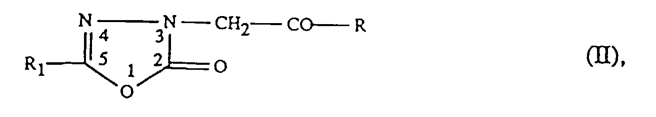 dans laquelle R et R₁ ont les significations données dans la revendication 1.
